# EUROPÄISCHE PATENTSCHRIFT

(11) **EP 0 533 708 B1**
(45) Veröffentlichungstag und Bekanntmachung des Hinweises auf die Patenterteilung: **22.02.1995**
(21) Anmeldenummer: 91910288.9
(22) Anmeldetag: 31.05.1991
(51) Int. Cl.: C07H 15/04

(54) **MODIFIZIERTES VERFAHREN ZUR DIREKTEN HERSTELLUNG VON ALKYLGLYKOSIDEN**
MODIFIED PROCESS FOR DIRECT PRODUCTION OF ALKYL GLYCOSIDES
PROCEDE MODIFIE POUR LA PREPARATION DIRECTE D'ALKYLGLUCOSIDES

(30) Priorität: 09.06.1990 DE 4018583
(43) Veröffentlichungstag der Anmeldung: 31.03.1993
(73) Patentinhaber: Henkel Kommanditgesellschaft auf Aktien, 40191 Düsseldorf (DE)
(72) Erfinder: WÜST, Willi, D-4030 Ratingen 6 (DE); WOLLMANN, Josef, D-5120 Herzogenrath 3 (DE); ESKUCHEN, Rainer, D-4000 Düsseldorf (DE)
(86) Internationale Anmeldenummer: EP9101010
(87) Internationale Veröffentlichungsnummer: WO9119722

(56) Entgegenhaltungen:
- EP-A- 0 096 917
- EP-A- 0 132 046
- EP-A- 0 362 671

## Beschreibung

Die Erfindung betrifft eine Weiterentwicklung der Herstellung von oberflächenaktiven Alkylglycosiden auf dem Wege der sogenannten Direktsynthese. In diesem Verfahren werden die Alkylglycoside, die bekanntlich Acetale aus Zuckern und monofunktionellen Alkoholen sind, durch direkte Säure-katalysierte Umsetzung der Alkohole mit den Zuckern unter Wasserabspaltung hergestellt.

Die vorliegende Erfindung betrifft insbesondere eine weitere Ausgestaltung eines Verfahrens der genannten Art, wie es beispielsweise in der Internationalen Patentanmeldung WO90/03977 ("Verfahren zur direkten Herstellung von Alkylglycosiden") beschrieben ist. In diesem Dokument wird der Begriff Alkylglycoside sowohl in seiner breiteren Fassung als insbesondere auch in der für praktische Einsatzzwecke engeren Form erläutert, wobei diese Angaben auch für die Lehre der vorliegenden Erfindung Gültigkeit haben. Die Zuckerkomponenten, die im nachfolgenden als Glycosen bezeichnet werden, umfassen Aldosen oder auch Ketosen im weitesten Sinne. Vorzugsweise werden wegen der besseren Reaktionsfähigkeit Aldosen verwendet. Hier kommt wegen der leichten Zugänglichkeit und Verfügbarkeit in technischen Mengen insbesondere die Glucose in Betracht, so daß insbesondere das Gebiet der Herstellung von oberflächenaktiven Alkylglucosiden betroffen ist. Der über die Acetalgruppierung an die Glucose gebundene Alkylrest leitet sich von monofunktionellen Alkoholen mit bevorzugt höherer Kettenlänge ab, wobei besondere Bedeutung entsprechenden Alkylresten mit 8 bis 20 Kohlenstoffatomen zukommen kann. Besonders geeignete Alkylreste leiten sich von Alkoholen ab, die aus Naturstoffen - beispielsweise Fetten und/oder Ölen natürlichen Ursprungs - gewonnen worden sind, wenn die Erfindung darauf allerdings auch keineswegs eingeschränkt ist. Der Begriff der Alkylglycoside umfaßt dabei Verbindungen der genannten Art, in denen die Alkylreste in Acetalform an einkernige und/oder an mehrkernige Zuckerreste gebunden sind.

Zu den Alkylglycosiden der genannten Art, ihrer Herstellung und ihrer Anwendung, insbesondere als oberflächenaktive Verbindungen besteht ein umfangreicher druckschriftlicher Stand der Technik, auf den in der genannten älteren Anmeldung der Anmelderin ausführlich eingegangen ist. Die zitierte Internationale Anmeldung WO90/03977 betrifft ihrerseits ein Verfahren zur direkten Herstellung von Alkylglycosiden durch Acetalisierungsreaktion von höheren aliphatischen primären Alkoholen mit Glycosen, insbesondere mit Glucose, in Gegenwart eines sauren Katalysators, rascher Entfernung des Reaktionswassers, Neutralisation des Katalysators mit einer Base, Abdestillation des Alkoholüberschusses und Überführen des Reaktionsproduktes in eine wäßrige Paste und Bleichen dieser Paste, wobei der aliphatische Alkohol im Überschuß zur Glycose eingesetzt wird, und wobei die Bildung und Abführung des Reaktionswassers unter Vakuum erfolgt und Reaktionstemperaturen oberhalb 80 °C angewendet werden. Das bekannte Verfahren ist im einzelnen dadurch gekennzeichnet, daß man a) Mischungen aus aliphatischem primären Alkohol, Glycose und saurem Katalysator bei erhöhter Temperatur erzeugt und zur Reaktion bringt, wobei man entweder i) eine Teilmenge des Alkohols mit dem Katalysator vorlegt, die Mischung erwärmt und dann eine erwärmte Suspension der Glycose in der restlichen Alkoholmenge portionsweise oder kontinuierlich zu der Alkohol/Katalysator-Mischung zudosiert und dabei im Vakuum das entstehende Reaktionswasser abdestilliert, oder ii) eine Mischung des gesamten Alkohols und der Glycose vorlegt, erwärmt und den sauren Katalysator zur erwärmten Mischung hinzugibt, dann ein Vakuum anlegt und weiter bis zum Einsetzen der Reaktion erwärmt und das entstehende Reaktionswasser abdestilliert, b) dabei die Ansatzverhältnisse so wählt, daß das Molverhältnis Glycose zu aliphatischem Alkohol bei 1 : 2 bis 1 : 10, vorzugsweise bei 1 : 3 bis 1 : 6 liegt, c) das Reaktionsgemisch bei dieser Temperatur und diesem Unterdruck so lange hält, vorzugsweise unter Durchmischung, bis das Reaktionswasser vollständig entfernt ist, d) anschließend das Reaktionsgemisch auf ca. 90 °C abkühlt, dann eine organische oder anorganische basische Alkali-, Erdalkali- oder Aluminium- bzw. Alkali/Aluminiumverbindung in solchen Mengen hinzufügt, daß sich über die Neutralisation des sauren Katalysators hinaus ein pH-Wert von wenigstens 8, vorzugsweise 8 bis 10 einstellt, und vorzugsweise erst danach Normaldruck herstellt, e) daß man vorzugsweise ohne vorherige Filtration den überschüssigen Alkohol aus dem alkalischen Gemisch im Vakuum auf an sich übliche, das Reaktionsprodukt schonende Weise auf einen Wert von unterhalb 5 Gew.-% des Reaktionsproduktes abdestilliert, und f) daß man an-schließend auf ca. 105 °C abkühlt und durch Zugabe von Wasser eine 30- bis 60%ige Paste erzeugt, die man durch vorzugsweise portionsweises Hinzufügen von Aktivsauerstoffverbindungen, vorzugsweise Wasserstoffperoxid, ca. 0,1 bis 5 Stunden bei ca. 80 °C rührt, wobei man gegebenenfalls durch Hinzufügen von Alkali, vorzugsweise Natronlauge, dafür sorgt, daß der pH-Wert während dieses Bleichprozesses bei 8 bis 10 bleibt. Bevorzugte höhere aliphatische primäre Alkohole für die Herstellung der Alkylglycoside enthalten 8 bis 20 Kohlenstoffatome, insbesondere 12 bis 18 Kohlenstoffatome.

Die zentrale Reaktion der Direktsynthese ist die sauer katalysierte Acetalisierung der eingesetzten Zucker, insbesondere der Glucose, mit den im Überschuß eingesetzten monofunktionellen Alkoholen. Zur Herstellung von hochwertigen Produkten einerseits der gewünschten vorgegebenen Konstitution, andererseits der Farbe bzw. Farbstabilität hat sich gezeigt, daß der praktischen Durchführung dieses entscheidenen Schrittes der Direktsynthese eine sehr starke Bedeutung zukommt. Schon die hier besprochene Internationale Anmeldung WO90/03977 geht darauf in mehrfacher Weise ein. Der hier betroffene Schritt a) des zuvor definierten Verfahrens sieht die zwei alternativen Möglichkeiten gemäß (a, i) bzw. gemäß (a, ii) vor. Die hier zuerst referierte Arbeitsweise hat den Vorteil, daß das bei der Acetalisierung entstehende Reaktionswasser und/oder das beispielsweise über den eindosierten Zuckerreaktanten - etwa Glucosemonohydrat - eingetragene Wasser rasch und kontinuierlich aus dem Reaktionsgemisch entfernt werden kann. Erreicht wird das dadurch, daß die Acetalisierung im Bereich eines niedrigen Vakuums von beispielsweise etwa 10 bis 50 mbar durchgeführt wird, so daß das Reaktionswasser bei den gleichzeitig eingesetzten hohen Reaktionstemperaturen oberhalb 100 °C, vorzugsweise im Bereich von etwa 120 °C über die Dampfphase abgeführt werden kann. Einer solchen kontinuierlichen Zugabe des Zuckerreaktanten im Verlaufe der Reaktion wird nach den Angaben des bekannten Verfahrens der Vorzug gegeben. Dabei soll zweckmäßigerweise die Dosierung in ihrer Geschwindigkeit so gewählt werden, daß im Reaktor ständig eine im wesentlichen klare Phase vorliegt, das heißt, daß die Menge der nicht umgesetzten Glycose im Reaktionsgemisch gering gehalten wird. Wichtig ist bei diesem bekannten Verfahren weiterhin, während der Reaktion die Mischung vorzugsweise ständig zu durchmischen und zu erwärmen, wobei es als wesentlich angesehen wird, daß zwischen Reaktorwand und Reaktionsgemisch nur eine geringe Temperaturdifferenz vorhanden ist, um auf diese Weise Überhitzungen zu vermeiden, die beispielsweise zu unerwünschten Farbverschlechterungen führen. Im einzelnen ist im Rahmen dieses bekannten Verfahrens dann angegeben, wie beispielsweise Reaktionstemperaturen von etwa 120 °C im Reaktor eingestellt und aufrechterhalten werden können, ohne daß Reaktorwandtemperaturen von mehr als etwa 125 °C eingesetzt werden müßten.

Die Lehre der vorliegenden Erfindung geht von der Aufgabe aus, für die Stufe der Acetalisierung - das heißt, für die Reaktionshauptstufe, die bei hohen Temperaturen und stark erniedrigten Drucken die eingespeisten Zucker mit den monofunktionellen Alkoholen zum Alkylglucosid umsetzt - nochmals verbesserte Arbeitsbedingungen anzugeben.

Gegenstand der vorliegenden Erfindung ist somit ein Verfahren zur Herstellung von Alkylglycosiden durch Direktsynthese aus höheren monofunktionellen Alkoholen, vorzugsweise Festalkoholen natürlichen Ursprungs mit 8 bis 20 und insbesondere 12 bis 18 C-Atomen, und pulverförmigen Glycosen in Gegenwart saurer Katalysatoren bei erhöhten Temperaturen, wobei der Reaktionsinnenraum unter vermindertem Druck gehalten und in den darin im Überschuß vorliegenden, den sauren Katalysator enthaltenden und auf Reaktionstemperatur erhitzten Alkoholreaktanten die Glycose zeitverzögert eindosiert wird, während im Reaktionsgemisch frei werdendes Wasser über die Gasphase aus dem Reaktionsraum abgezogen wird. Das neue Verfahren ist dadurch gekennzeichnet, daß aus der heißen flüssigen Reaktionsmischung ein Teilstrom abgezogen und einer Vormischzone zugeführt wird, in die gleichzeitig der pulverförmige Reaktant eingespeist und hier mit dem flüssigen Teilstrom zu einer Paste aufbereitet und über einen nachgeschalteten Intensivmischer dem Reaktorinneren zugeführt wird, wobei diese Vormischzone über den Intensivmischer mit dem verminderten Druck des Reaktorinneren und gleichzeitig über die Fördervorrichtung der pulverförmigen Glycose mit dem Umgebungsdruck in unmittelbarem Druckausgleich steht und daß dabei weiterhin die Konsistenz der in der Vormischzone gebildeten Paste derart gewählt wird, daß diese Paste als Dichtmasse zum Druckausgleich dient.

Die erfindungsgemäße Lehre arbeitet also insbesondere dergestalt, daß der im Überschuß eingesetzte Alkoholreaktant als Ganzes in den Reaktor vorgegeben werden kann und dort auf Reaktionstemperatur erhitzt und mit dem sauren Katalysator vermischt werden kann. Im erfindungsgemäßen Verfahren ist es somit möglich, die als Feststoff, insbesondere in Pulverform eingesetzte Glykose unter Einhaltung der Reaktionsbedingungen von Temperatur und Vakuum vorzugsweise kontinuierlich in den Reaktor einzudosieren, wobei die Geschwindigkeit dieser Zudosierung dem Reaktionsfortschritt derart angepaßt werden kann, daß eine jeweils optimale Steuerung in der Beschaffenheit des Reaktorinhaltes und insbesondere des hier jeweils gegebenen Umsetzungsgrades gewährleistet sind.

Die technische Lösung der erfindungsgemäßen Aufgabe macht also von dem Merkmal Gebrauch, Anteile der vorgelegten bzw. jeweils vorliegenden fließfähigen Phase aus dem Reaktorinhalt unter Verfahrensbedingungen abzuziehen und einer Vormischzone zuzuführen, in die gleichzeitig der Glykosereaktant in Feststoff-Form - vorzugsweise als Pulver - eindosiert wird. In dieser Vormischzone wird aus der im Kreislauf geführten Flüssigphase und der zugeführten Feststoffphase eine Paste gebildet, deren Beschaffenheit so eingestellt wird, daß diese Paste als sogenannte "lebende Dichtung'' fungieren kann. Trotz bevorzugt kontinuierlichen Durchsatzes des einzuspeisenden Feststoffreaktanten in das bei niedrigem Druck gehaltene Reaktorinnere ist gleichwohl der vorgegebene stark abgesenkte Druck im Reaktorinneren zuverlässig einstellbar, gleichzeitig ist über die Geschwindigkeit der Einspeisung der Paste in das Reaktorinnere der Reaktionsablauf im Reaktorinneren und damit die Beschaffenheit der hier gegebenen Flüssigphase genau steuerbar. Nach Abschluß der Einspeisung des Feststoffreaktanten in Pastenform kann der Reaktionsraum durch geeignete mechanische Elemente, beispielsweise Schieber, bleibend verschlossen und damit dann die Phase der Nachreaktion bei bevorzugt weiterhin zunehmend erniedrigten Drucken durchgeführt werden.

Wie im eingangs zitierten Verfahren des Standes der Technik wird auch nach dem erfindungsgemäßen Verfahren mit Reaktorinnendrucken im Bereich unterhalb 100 mbar und vorzugsweise während der Phase der Glycosezudosierung im Bereich von etwa 10 bis 50 mbar gearbeitet. Zwischen dem Reaktorinneren und dem normalen Außendruck, der üblicherweise leicht über 1000 mbar liegt, besteht also eine beträchtliche Druckdifferenz. Die als lebende Dichtmasse fungierende Paste muß in ihrer Beschaffenheit deshalb so eingestellt werden, daß sie - im Zusammenwirken mit der beispielsweise über eine Förderschnecke zugeführten pulverförmigen Glycose - unter den jeweils gegebenen Verhältnissen als wirkliches Abdichtelement gegen den Einbruch des weitaus höheren Außendruckes in das Reaktorinnere dienen kann.

Zur Herstellung der Paste wird der der Vormischzone zugeführte Flüssiganteil in diese normalerweise mit beschränkt erhöhtem Druck eingespeist. Hier besteht die Gefahr, daß bei Wahl zu hoher Flüssigdrucke Anteile der Flüssigphase durch die von Außen, vorzugsweise kontinuierlich zugeförderte pulverförmige Glucose durchbricht. Hier liegt also eine zweite Gefahrenquelle für den angestrebten störungsfreien, bevorzugt kontinuierlichen Verfahrensabschnitt der zeitlich gesteuerten Eindosierung des Feststoffreaktanten in das Reaktorinnere.

Zum Ausschluß von Störungen der zuletzt dargestellten Art sieht das erfindungsgemäße Verfahren in der bevorzugten Ausführungsform vor, daß auch in der Vormischzone bei Unterdruck gearbeitet wird. Die hier eingestellten Unterdrucke sind jetzt aber im Vergleich mit dem im Reaktionsinneren vorgelegten Unterdruck abgemildert und liegen insbesondere dem Außendruck näher als dem stark abgesenkten Innendruck. Vorzugsweise wird der Druck im Bereich der Vormischzone bei etwa 300 bis 900 mbar, insbesondere bei etwa 500 bis 900 mbar gehalten; besonders bevorzugt ist der Bereich von etwa 400 bis 850 mbar und ganz besonders der von etwa 750 bis 850 mbar. Die Einstellung dieses Druckbereiches gelingt durch angemessene Steuerung der pastenbildenden flüssigen und festen Materialströme zur Vormischzone und deren bevorzugt kontinuierlichen Abführung in das Reaktorinnere. Zur Ausbildung der in der Vormischzone als lebende Dichtung fungierenden Pastenbeschaffenheit haben sich ausgewählte Massenverhältnisse der Ströme von flüssigem und festen Reaktanten als vorteilhaft erwiesen. Die bevorzugten Massenverhältnisse der Flüssigphase zu der Masse des eingeführten Glycosereaktanten liegen im Bereich von etwa 20 bis 200 zu 1.

Bereits in der eingangs zitierten Internationalen Anmeldung wird darauf verwiesen, daß die Feindispergierung der Glycose im erhitzten und Katalysator enthaltenden flüssigen Alkoholreaktanten einen wesentlichen positiven Einfluß auf die Qualität des Reaktionsendproduktes hat. Es wird daher vorgeschlagen - und davon macht auch die Lehre der Erfindung in ihrer bevorzugten Ausführungsform Gebrauch - die primär entstehende Wirkstoffmischung durch geeignete technische Feinstmischelemente einer Feindispergierung zu unterwerfen. Als besonders geeignet haben sich hier sogenannte Inline-Mischer beispielsweise vom Stator/Rotor-Prinzip bewährt. Diese Feindispergierung hat den erwünschten Nebeneffekt, daß sich die Paste bei der Verarbeitung erwärmt. Im einzelnen ist dann die durch das erfindungsgemäße Verfahren betroffene Einheit der Reaktoranlage beispielsweise wie folgt ausgebildet:
Von der Hauptreaktion getrennt, aber in unmittelbarer Druckanbindung an die Hauptreaktionszone ist eine Vormischzone vorgesehen. Dieser Vormischzone wird einerseits der trockene, bevorzugt pulverförmige Glycosereaktant beispielsweise durch eine Förderschnecke zugeführt. Gleichzeitig wird über eine Zweigleitung aus dem Inneren des Reaktors Flüssigphase in Abstimmung mit der eingespeisten festen Reaktantenmenge so der Vormischzone zugegeben, daß hier die Paste als lebende Dichtung - insbesondere im Bereich der angegebenen Massenverhältnisse von Flüssigphase zu Feststoffphase - ausgebildet wird. Aus der Vormischzone wandert die Paste im Sinne des Druckgefälles in Richtung auf das Reaktorinnere in den Intensivmischer, beispielsweise also in den nachgeschalteten Inline-Mischer und tritt aus diesem dann in das Reaktorinnere ein. Die Geschwindigkeit des Materialdurchtritts durch die Vormischzone kann unter diesen Bedingungen praktisch beliebig gesteuert und dem Reaktionsablauf im Reaktorinneren optimal angepaßt werden. Es ist eine absatzweise und/oder kontinuierliche Zugabe des Feststoffreaktanten möglich, wobei die kontinuierliche zeitverzögerte Zugabe üblicherweise bevorzugt ist.

Nach Abschluß der vorgegebenen Menge des Feststoffreaktanten wird der Reaktionsinnenraum von der Umgebung abgeschlossen. Möglich ist das beispielsweise durch einen in dem Bereich der Vormischzone vorgesehenen Schieber, der etwa die Vormischzone und die Förderschnecke für das Feststoffgut voneinander trennt. Während der Einspeisung der Glycose ist der Schieber geöffnet. Nach Zugabe der insgesamt benötigten Menge des Feststoffreaktanten wird der Schieber geschlossen. Jetzt kann die Acetalisierungsreaktion im Reaktorinneren fortgeführt und in der Weise abgeschlossen werden, wie es im einzelnen im Rahmen der zitierten Internationalen Anmeldung beschrieben ist. Nach einer angemessenen Nachreaktionszeit kann der Anlagendruck weiter abgesenkt werden und vorzugsweise auf Enddrucke im Bereich von einigen mbar, beispielsweise auf Werte von 2 bis 5 mbar abgesenkt werden. Anschließend wird das Reaktionsgemisch beschränkt abgekühlt - beispielsweise auf Werte von etwa 90 °C - dann wird der Katalysator neutralisiert, wobei vorzugsweise basische pH-Werte von wenigstens pH 8, insbesondere von pH 8 bis 10, eingestellt werden, woraufhin der überschüssige Alkohol aus dem alkalisierten Gemisch im Vakuum auf an sich übliche, das Reaktionsprodukt schonende Weise auf geringe Restwerte abdestilliert wird. Schließlich wird in der bereits angegebenen Weise die Bleiche des Reaktionsrohprodukts durchgeführt.

Geeignete saure Katalysatoren sind beispielsweise Schwefelsäure, Phosphorsäure, Sulfobernsteinsäure, aliphatische und/oder aliphatisch-aromatische Sulfonsäuren mit 1 bis 16 C-Atomen im aliphatischen Rest, beispielsweise Paratoluolsulfonsäure und dergleichen, die üblicherweise in Mengen von etwa 0,05 bis 0,02 Mol pro Mol der eingesetzten Glycose zum Einsatz kommen. Als basische Substanzen für die Neutralisation des sauren Katalysators und darüber hinaus für die Einstellung eines basischen pH-Wertes werden anorganische feinpulverisierte Verbindungen aus der Gruppe Calciumhydroxid, Calciumoxid, Magnesiumhydroxid, Magnesiumoxid, die Zeolithe NaA oder NaX, vorzugsweise in Kombination mit Calciumhydroxid und/oder als organische Verbindungen die Alkoholate von niedrig-siedenden Alkoholen mit vorzugsweise 1 bis 4 C-Atomen in Form ihrer Alkalimetall- und/oder Erdalkalimetallverbindungen bevorzugt. Besondere Bedeutung kommt dem Magnesiumoxid bzw. den Magnesiumalkoholaten, beispielsweise dem Magnesiumethylat zu. Diese basischen Magnesiumverbindungen können auch beim Neutralisieren des Katalysators und dem darüber hinausgehenden Einstellen eines basischen pH-Wertes von 8 bis 10 zur Hälfte bis zu dreiviertel der äquivalenten Mengen durch Alkalihydroxid oder -carbonat, insbesondere NaOH ersetzt werden (also 2 Mol basische Na-Verbindungen pro 1 Mol basische Mg-Verbindung). Zur Abdestillation des Alkoholüberschusses wird unter einem Vakuum gearbeitet, das Sumpftemperaturen von 160 bis 170 °C ermöglicht, wobei die Abdestillation des Alkoholüberschusses in Kurzwegverdampfern besonders geeignet ist. Für technische Ansätze sind besonders die Fallfilmverdampfer oder die Dünnschichtverdampfer geeignet.

Die Reaktionsprodukte der Erfindung sind Gemische aus Alkylmonoglycosid und den Oligomerisierungsprodukten mit einem Oligomerisierungsgrad bis etwa 5. Vorzugsweise liegt der durchschnittliche Oligomerisierungsgrad bei maximal etwa 1,5, insbesondere 1,2 bis 1,4. Die Reaktion wird insbesondere so geführt wird, daß die Menge an Alkylmonoglucosid bezogen auf die Gesamtmenge aus Alkylmonoglucosid und Alkyloligoglucosid deutlich über 70 Gew.-% liegt. Dazu wird vorzugsweise mit Molverhältnissen von Glykose/höherem Alkohol im Bereich von 1:2 bis 1:10, insbesondere von 1:3 bis 1:6 gearbeitet. Die Restalkoholmengen bezogen auf wasserfreies Produkt sollten höchstens etwa 5 Gew.-% betragen und liegen insbesondere im Bereich von etwa 0,5 bis 2,5 Gew.-%. Aus den Reaktionsprodukten können wäßrige Pasten mit 30 bis 60 Gew.-% Wasseranteil gebildet werden, wobei diese Pasten auch die aus der Neutralisation des Katalysators und dem Bleichvorgang stammenden Salze enthalten. Durch Zusatz von antimikrobiellen Wirkstoffen in geringen Mengen kann die Lagerungsbeständigkeit der Alkylglycoside verbessert werden.

Zur Herstellung einer waschaktiven Alkylglucosidverbindung wurden in einem mit Rührwerk versehenen Reaktionsbehälter (2,5 m³) die nachfolgenden Reaktanten zur Umsetzung gebracht: 1213 kg C₁₂₋₁₄-Fettalkohol (Handelsprodukt Lorol Spezial der Anmelderin), 250 kg wasserfreie Glukose bzw. - in einem Parallelversuch - 275 kg Glukosemonohydrat, 1,1 kg Sulfobernsteinsäure als Katalysator, 1,3 kg Magnesiumethylat als Neutralisationsmittel.

Die zur Reaktion eingesetzte Anlage war wie folgt ausgestaltet: Über ein im Boden des Reaktionsbehälters angeordnetes Ventil konnten Anteile des fließfähigen Reaktorinhalts über eine Kreislaufleitung abgezogen und am Kopf des Reaktionsbehälters wieder eingespeist werden. Der Flüssigkeitstransport wurde mit einer im Kreislauf angeordneten Pumpe vorgesehen. Die Kreislaufleitung war beheizt. Der abgezogene Flüssigkeitsteilstrom wurde einem außerhalb des Reaktionsbehälters angeordneten Mischelement zugeführt, das über einen Intensivmischer (Inline-Mischer) mit dem Inneren des Reaktionsbehälters verbunden war, während gleichzeitig mittels einer Förderschnecke der pulverförmige Glukosereaktant in das Mischelement eingespeist werden konnte. Im einzelnen wurde wie folgt gearbeitet:

Die Gesamtmenge des Fettalkohols und der Katalysator wurden in den Reaktor eingegeben und auf die Reaktionstemperatur von 110 bis 120 °C unter gleichzeitiger Umwälzung über das Kreislaufsystem aufgeheizt. Der Anlagendruck wurde auf 10 bis 20 mbar reduziert. Während dieser Anlaufphase war die Förderschnecke für die pulverförmige Glukose mittels eines verschlossenen Schiebers vom System abgetrennt.

Nach Erreichen der angegebenen Werte für die Reaktionstemperatur und den Solldruck erfolgte durch Einschalten des Inline-Mischers und des Schneckenantriebs und durch Öffnen des Flachschiebers die Inbetriebnahme der Glukoseeinspeisung. Bei einer Kreislauffördermenge von 10 m³/h des flüssigen Reaktorinhalts wurden über die Mischvorrichtung in einem ersten Versuchsansatz innerhalb eines Zeitraums von ca. 40 Minuten 250 kg wasserfreie Glukose eingespeist, während in einem parallelen zweiten Versuchsansatz die 275 kg Glukosemonohydrat in ca. 75 Minuten eindosiert wurden. Die aus dem Reaktor austretenden Brüden (Destillate) wurden in einem ersten Kondensator (Warmwasser von 25 °C) von mitgetragenen Fettalkoholanteilen befreit, in einem zweiten Kondensator wurde das Reaktionswasser niedergeschlagen.

Nach Beendigung der Glukosezudosierung schloß sich in beiden Fällen eine Nachreaktionszeit von 1,5 h an. Innerhalb dieser Nachreaktionszeit wurde der Anlagendruck unter Verwendung eines zweistufigen Dampfstrahlers auf ca. 2 bis 3 mbar reduziert.

Die Neutralisation erfolgte mit Magnesiumethylat unter Vakuum. Nach Einstellung eines pH-Wertes oberhalb von 8 wird das Reaktionsgemisch in einen Zwischenlagertank übergeführt. Nachfolgend wurde die destillative Abtrennung des Fettalkoholüberschusses vorgenommen. Die angefallene Alkylglykosidverbindung wurde in an sich bekannter Weise der Bleiche mit beispielsweise Wasserstoffperoxid zugeführt.

## Patentansprüche

1. Verfahren zur Herstellung von Alkylglycosiden durch Direktsynthese aus höheren monofunktionellen Alkoholen und pulverförmigen Glycosen in Gegenwart saurer Katalysatoren bei erhöhten Temperaturen, wobei der Reaktionsinnenraum unter vermindertem Druck gehalten und in den darin im Überschuß vorliegenden, den sauren Katalysator enthaltenden und auf Reaktionstemperatur erhitzten Alkoholreaktanten die Glycose zeitverzögert eindosiert wird, während im Reaktionsgemisch frei werdendes Wasser über die Gasphase aus dem Reaktionsraum abgezogen wird, dadurch gekennzeichnet, daß aus der heißen flüssigen Reaktionsmischung ein Teilstrom abgezogen und einer Vormischzone zugeführt wird, in die gleichzeitig der pulverförmige Reaktant eingespeist und hier mit dem flüssigen Teilstrom zu einer Paste aufbereitet und über einen nachgeschalteten Intensivmischer dem Reaktorinneren zugeführt wird, wobei diese Vormischzone über den Intensivmischer mit dem verminderten Druck des Reaktorinneren und gleichzeitig über die Fördervorrichtung der pulverförmigen Glycose mit dem Umgebungsdruck in unmittelbarem Druckausgleich steht und daß dabei weiterhin die Konsistenz der in der Vormischzone gebildeten Paste derart gewählt wird, daß diese Paste als Dichtmasse zum Druckausgleich dient.

2. Verfahren nach Anspruch 1, dadurch gekennzeichnet, daß mit Reaktorinnendrucken im Bereich unterhalb 100 mbar und vorzugsweise während der Phase Glycose-Zudosierung im Bereich von etwa 10 bis 50 mbar gearbeitet wird.

3. Verfahren nach Ansprüchen 1 und 2, dadurch gekennzeichnet, daß auch in der Vormischzone bei Unterdruck gearbeitet wird, der im Vergleich mit dem Reaktorinneren jedoch abgemildert ist und vorzugsweise im Bereich von etwa 300 bis 900 mbar und insbesondere im Bereich von etwa 500 bis 900 mbar liegt.

4. Verfahren nach Ansprüchen 1 bis 3, dadurch gekennzeichnet, daß in der Paste der Vormischzone Massenverhältnisse der Flüssigphase zum Glycosereaktanten im Bereich von 20 bis 200 zu 1 eingestellt werden.

5. Verfahren nach Ansprüchen 1 bis 4, dadurch gekennzeichnet, daß wasserfreie Glucose und/oder Glucosemonohydrat als pulverförmiger Reaktant eingespeist werden.

6. Verfahren nach Ansprüchen 1 bis 5, dadurch gekennzeichnet, daß nach Abschluß der Glucosezugabe der Reaktionsinnenraum verschlossen und das Reaktionsgemisch vorzugsweise unter weiter erniedrigtem Reaktorinnendruck einer Nachreaktion unterworfen wird, wobei der Anlagendruck auf bevorzugte Enddrucke im Bereich von einigen mbar, z. B. bei 2 bis 5 mbar abgesenkt werden kann.

7. Verfahren nach Ansprüchen 1 bis 6, dadurch gekennzeichnet, daß die in der Vormischzone gebildete und dort als lebende Dichtung dienende Paste über einen Inline-Mischer dem Reaktorinnenraum zugeführt wird.

8. Verfahren nach Ansprüchen 1 bis 7, dadurch gekennzeichnet, daß der pulverförmige Glycosereaktant der Vormischzone mit einer Förderschnecke zudosiert wird.

9. Verfahren nach Ansprüchen 1 bis 8, dadurch gekennzeichnet, daß mit Molverhältnissen von Glycose/höherem Alkohol im Bereich von 1 : 2 bis 1 : 10, insbesondere von 1 : 3 bis 1 : 6 gearbeitet wird.

10. Verfahren nach Ansprüchen 1 bis 9, dadurch gekennzeichnet, daß Fettalkohole, vorzugsweise natürlichen Ursprungs, mit 8 bis 20 C-Atomen, vorzugsweise mit 12 bis 18 C-Atomen zur Direktsynthese eingesetzt werden.

## Claims

1. A process for the production of alkyl glycosides by direct synthesis from higher monofunctional alcohols and powder-form glycoses in the presence of acidic catalysts at elevated temperatures, the interior of the reactor being kept under reduced pressure and the glycose being introduced with delay into the alcohol reactants present in excess therein, the alcohol reactants containing the acidic catalyst and being heated to the reaction temperature, while water released in the reaction mixture is removed from the reaction zone via the gas phase, characterized in that a partial stream is run off from the hot liquid reaction mixture and delivered to a premixing zone into which the powder-form reactant is simultaneously introduced and is converted with the liquid partial stream into a paste which is delivered to the interior of the reactor via a following intensive mixer, the premixing zone being in direct pressure equalization with the reduced pressure of the reactor interior via the intensive mixer and, at the same time, with the ambient pressure via the feed unit used for the powder-form glycose, and in that the consistency of the paste formed in the premixing zone is selected so that the paste serves as a pressure-equalizing sealing compound.

2. A process as claimed in claim 1, characterized in that internal reactor pressures below 100 mbar and in the range from about 10 to 50 mbar, preferably during the glycose addition phase, are applied.

3. A process as claimed in claims 1 and 2, characterized in that a reduced pressure is also applied in the premixing zone, but is lower than that applied in the interior of the reactor, preferably being in the range from about 300 to 900 mbar and more particularly in the range from about 500 to 900 mbar.

4. A process as claimed in claims 1 to 3, characterized in that mass ratios of the liquid phase to the glycose reactant of 20 to 200:1 are adjusted in the paste of the premixing zone.

5. A process as claimed in claims 1 to 4, characterized in that anhydrous glucose and/or glucose monohydrate is/are fed in as the powder-form reactant.

6. A process as claimed in claims 1 to 5, characterized in that, after the glucose has been added, the interior of the reactor is closed and the reaction mixture is subjected to an after-reaction, preferably under a further reduced internal reactor pressure, the plant pressure being reduced to preferred final pressures of a few mbar, for example from 2 to 5 mbar.

7. A process as claimed in claims 1 to 6, characterized in that the paste formed in the premixing zone, where it acts as a living seal, is delivered to the interior of the reactor via an inline mixer.

8. A process as claimed in claims 1 to 7, characterized in that the powder-form glycose reactant is introduced into the premixing zone by means of a screw conveyor.

9. A process as claimed in claims 1 to 8, characterized in that molar ratios of glycose to higher alcohol of 1:2 to 1:10 and, more particularly, 1:3 to 1:6 are applied.

10. A process as claimed in claims 1 to 9, characterized in that C₈₋₂₀ and preferably C₁₂₋₁₈ fatty alcohols, preferably of natural origin, are used for the direct synthesis.

## Revendications

1. Procédé de préparation d'alkylglucosides par synthèse directe à partir d'alcools monofonctionnels et de glucose en poudre en présence de catalyseurs acides à des températures élevées, l'espace intérieur réactionnel étant maintenu sous pression réduite et le glucose étant introduit par dosage différé dans le temps dans les réactants alcool présents en excès, contenant le catalyseur acide et chauffé à température de réaction, tandis que dans le mélange réactionnel l'eau se libérant est retirée de l'espace réactionnel par la phase gazeuse, caractérisé en ce qu'un courant partiel est retiré du mélange réactionnel liquide très chaud et est amené à une zone de prémélange, dans laquelle en même temps est alimenté le réactant en poudre et à ce moment il est préparé avec le courant partiel liquide en une pâte et est amené par l'intermédiaire d'un mélangeur intensif monté à la suite à l'intérieur du réacteur, tandis que cette zone de prémélange se trouve en équilibrage de pression direct par le mélangeur intensif avec la pression réduite de l'intérieur du réacteur et en même temps par le dispositif de transport du glucose en poudre avec la pression environnante et en ce qu'en outre la consistance de la pâte formée dans la zone de prémélange est choisie de manière que cette pâte serve comme pâte d'étanchéité pour l'équilibrage de pression.

2. Procédé selon la revendication 1, caractérisé en ce qu'on opère avec des pressions intérieures du réacteur dans la plage en dessous de 100 mbars et de préférence pendant la phase d'addition dusée du glucose dans la plage d'environ 10 à 50 mbars.

3. Procédé selon les revendications 1 et 2, caractérisé en ce que dans la zone de prémélange on opère à une pression diminuée adoucie toutefois en comparaison avec l'intérieur du réacteur et qui se situe de préférence dans la plage environ de 300 à 900 mbars, en particulier de 500 à 900 mbars.

4. Procédé selon les revendications 1 à 3, caractérisé en ce qu'on ajuste dans la pâte de la zone de prémélange des rapports massiques de la phase liquide au réactant glucose compris dans la plage de 20 à 200:1.

5. Procédé selon les revendication 1 à 4, caractérisé en ce qu'on introduit du glucose anhydre et/ou du monohydrate de glucose comme réactant pulvérulent.

6. Procédé selon les revendications 1 à 5, caractérisé en ce qu'après achèvement de l'addition de glucose l'espace intérieur réactionnel est fermé et le mélange réactionnel est soumis de préférence à une réaction ultérieure sous une pression intérieure du réacteur abaissée à nouveau, la pression de l'installation pouvant être abaissée à des pressions finales préférentielles au niveau de quelques mbars, par exemple de 2 à 5 mbars.

7. Procédé selon les revendications 1 à 6, caractérisé en ce que la pâte formée dans la zone de prémélange et servant là de garniture d'étanchéité vivante est amenée à l'espace intérieur du réacteur par un mélange "inline".

8. Procédé selon les revendications 1 à 7, caractérisé en ce que le réactant glucose en poudre est ajouté en dosant à la zone de prémélange par une vis sans fin.

9. Procédé selon les revendications 1 à 8, caractérisé en ce qu'on opère avec des rapports molaires de glucose/alcool supérieur dans la plage de 1:2 à 1:10, en particulier de 1:3 à 1:6.

10. Procédé selon les revendications 1 à 9, caractérisé en ce que des alcools gras, d'origine naturelle, avec de 8 à 20 atomes de C, de préférence avec de 12 à 18 atomes de C sont mis en oeuvre pour la synthèse directe.
